(19) Europäisches Patentamt
European Patent Office
Office européen des brevets

(11) **EP 2 556 819 A1**

(12) **EUROPEAN PATENT APPLICATION**
published in accordance with Art. 153(4) EPC

(43) Date of publication:
**13.02.2013 Bulletin 2013/07**

(21) Application number: **11765998.7**

(22) Date of filing: **07.04.2011**

(51) Int Cl.:
***A61K 8/34*** (2006.01) ***A61Q 19/02*** (2006.01)

(86) International application number:
**PCT/JP2011/058854**

(87) International publication number:
**WO 2011/126098 (13.10.2011 Gazette 2011/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2010 JP 2010089775**

(71) Applicant: **Kao Corporation**
**Chuo-Ku**
**Tokyo 103-8210 (JP)**

(72) Inventors:
- **SATOU, Kouji**
  **Odawara-shi**
  **Kanagawa 250-0002 (JP)**
- **SASAKI, Minoru**
  **Odawara-shi**
  **Kanagawa 250-0002 (JP)**

(74) Representative: **HOFFMANN EITLE**
**Patent- und Rechtsanwälte**
**Arabellastraße 4**
**81925 München (DE)**

(54) **EXTERNAL SKIN PREPARATION**

(57) A compound having a good whitening action as well as stability, an interaction with a base material for a composition for external use, and the like is searched for, and a composition for external use on skin containing the compound is provided. The composition for external use on skin contains a compound represented by the following formula (1):

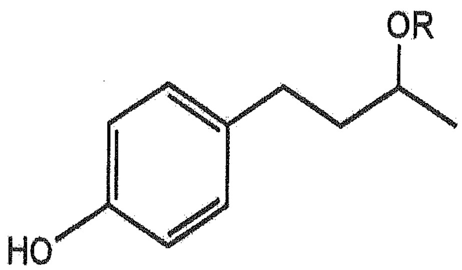

(1)

(wherein R represents an alkyl group having 1 to 8 carbon atoms).

EP 2 556 819 A1

## Description

[Field of the Invention]

The present invention relates to a melanin production inhibitor and a composition for external use on skin containing the melanin production inhibitor.

[Background of the Invention]

Age spots and freckles are excessive accumulation of melanin in the epidermal cells as a result of unbalance between the generation and elimination of melanin. These occur due to various causes including inflammation, balance of hormones, genetic factors, and the like, and they are promoted by the influence of ultraviolet rays. Whitening agents relieve the increased pigmentation.
Among them, as whitening agents applied to whitening cosmetics which prevent pigmentation of the skin or age spots and freckles and which keeps the originally white skin, kojic acid, arbutin, hydroquinone monobenzyl ether, hydrogen peroxide, and the like, are known. However, when these whitening agents are blended in the cosmetics, some hypochromic effects to pigmented skin are observed, but sufficient effects as whitening cosmetics cannot be exhibited. Furthermore, an inflammation suppressing effect due to ultraviolet rays is not exhibited, and there have been problems in terms of safety. Furthermore, as whitening agents for suppressing inflammation by ultraviolet rays, vitamin C and derivatives thereof are known.
Furthermore, raspberry ketone, rhododendrol or derivatives thereof is known to have excellent melanin production-inhibitory and whitening actions (see Patent Document 1).

[Prior art Document]

[Patent Document]

[Patent Document 1] JP-A-10-265325
[Patent Document 2] JP-A-2-188547

[Summary of the Invention]

[Technical Problem]

However, these whitening ingredients have disadvantages such as an insufficient whitening effect, insufficient solubility in a base material for a composition for external use, and insufficient stability in formulations.
Therefore, the present invention relates to searching for a compound which has, in addition to a good whitening action, excellent stability and solubility in a base material for a composition for external use to provide a composition for external use on skin containing the compound.

[Solution to Problem]

The present inventors have keenly studied for solving the above-mentioned problems, and resulted in finding that a compound represented by the following formula (1) has more excellent whitening effect than that of existing whitening agents, for example, more excellent than a compound described in Patent Document 1, and the present inventors also have found that the compound can stably be blended in various formulation systems because it does not have an unstable molecular structure in its molecules, and it is excellent in solubility in a base material for a composition for external use, and thus they have completed the present invention.
That is to say, the present invention provides the inventions of the following [1] to [30].
[1] A composition for external use on skin containing a compound represented by the following formula (1).

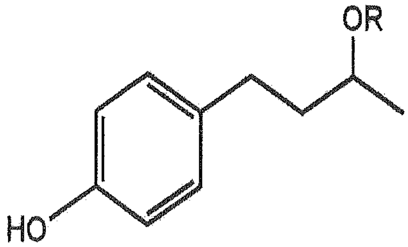

(1)

(In the formula, R represents an alkyl group having 1 to 8 carbon atoms.)

[2] The composition for external use on skin according to [1], wherein R in the formula (1) is an alkyl group having 1 to 4 carbon atoms.
[3] The composition for external use on skin according to [1], wherein R in the formula (1) is a methyl group, an ethyl group, a n-propyl group, or an isopropyl group.
[4] The composition for external use on skin according to [1], wherein R in the formula (1) is an ethyl group.
[5] A whitening cosmetic containing a compound represented by the following formula (1).

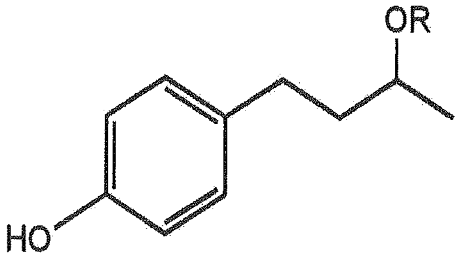

(1)

(In the formula, R represents an alkyl group having 1 to 8 carbon atoms.)

[6] The whitening cosmetic according to [5], wherein R in the formula (1) is an alkyl group having 1 to 4 carbon atoms.
[7] The whitening cosmetic according to [5], wherein R in the formula (1) is a methyl group, an ethyl group, a n-propyl group, or an isopropyl group.
[8] The whitening cosmetic according to [5], wherein R in the formula (1) is an ethyl group.
[9] A melanin production inhibitor containing a compound represented by the following formula (1) as an active ingredient.

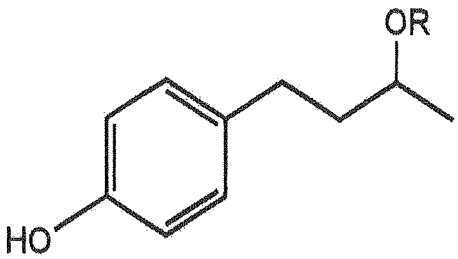

(1)

(In the formula, R represents an alkyl group having 1 to 8 carbon atoms.)

[10] The melanin production inhibitor according to [9], wherein R in the formula (1) is an alkyl group having 1 to 4 carbon atoms.
[11] The melanin production inhibitor according to [9], wherein R in the formula (1) is a methyl group, an ethyl group, a n-propyl group, or an isopropyl group.
[12] The melanin production inhibitor according to [9], wherein R in the formula (1) is an ethyl group.

[13] A compound represented by the following formula (1) for external use on skin.

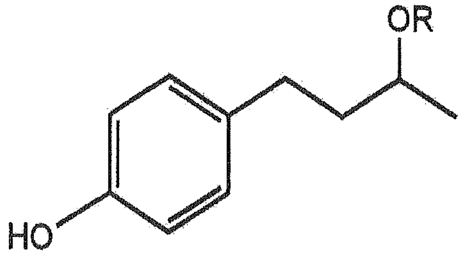

(1)

(In the formula, R represents an alkyl group having 1 to 8 carbon atoms.)

[14] The compound according to [13], wherein R in the formula (1) is an alkyl group having 1 to 4 carbon atoms.
[15] The compound according to [13], wherein R in the formula (1) is a methyl group, an ethyl group, a n-propyl group, or an isopropyl group.
[16] The compound according to [13], wherein R in the formula (1) is an ethyl group.
[17] The compound according to any one of [13] to [16], wherein the external use on skin is application to the skin for whitening.
[18] The compound according to any one of [13] to [16], wherein the external use on skin is application to the skin for inhibiting production of melanin in the skin.
[19] Use of a compound represented by the following formula (1) for manufacturing a composition for external use on skin.

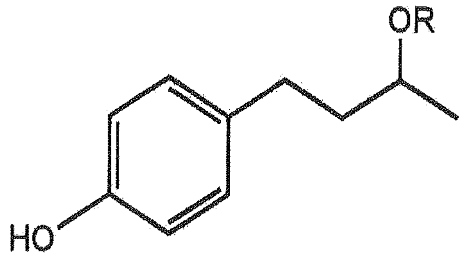

(1)

(In the formula, R represents an alkyl group having 1 to 8 carbon atoms.)

[20] The use according to [19], wherein R in the formula (1) is an alkyl group having 1 to 4 carbon atoms.
[21] The use according to [20], wherein R in the formula (1) is a methyl group, an ethyl group, a n-propyl group, or an isopropyl group.
[22] The use according to [20], wherein R in the formula (1) is an ethyl group.
[23] Use of a compound represented by the following formula (1) for manufacturing a whitening cosmetic.

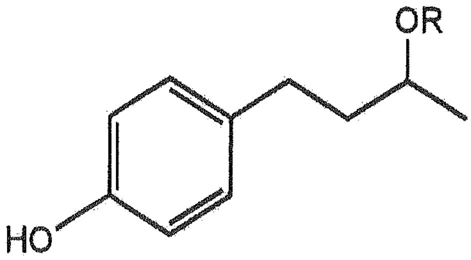

(1)

(In the formula, R represents an alkyl group having 1 to 8 carbon atoms.)

[24] The use according to [23], wherein R in the formula (1) is an alkyl group having 1 to 4 carbon atoms.
[25] The use according to [23], wherein R in the formula (1) is a methyl group, an ethyl group, a n-propyl group, or an isopropyl group.
[26] The use according to [23], wherein R in the formula (1) is an ethyl group.
[27] Use of a compound represented by the following formula (1) for manufacturing a melanin production inhibitor.

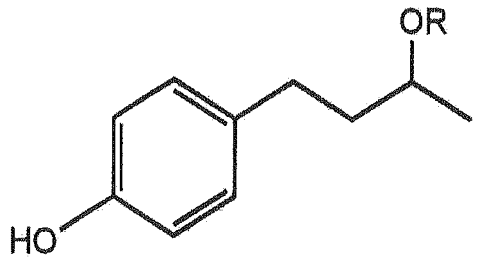

(1)

(In the formula, R represents an alkyl group having 1 to 8 carbon atoms.)

[28] The use according to [27], wherein R in the formula (1) is an alkyl group having 1 to 4 carbon atoms.
[29] The use according to [27], wherein R in the formula (1) is a methyl group, an ethyl group, a n-propyl group, or an isopropyl group.
[30] The use according to [27], wherein R in the formula (1) is an ethyl group.

[Effects of the Invention]

Since the compound (1) used in the present invention has a good whitening action, is excellent in stability, and has good solubility in a base material for a composition for external use, it can easily be applied to various compositions for external use, and can provide a composition for external use on skin having a good whitening action. Furthermore, the composition for external use on skin is also excellent in a feeling upon use and preference.

[Brief Description of the Drawings]

[Fig. 1] Fig 1 is a graph showing a $^{13}C$-NMR spectrum of 4-(3-methoxybutyl)phenol in Production Example 1.
[Fig. 2] Fig 2 is a graph showing a $^{13}C$-NMR spectrum of 4-(3-ethoxybutyl)phenol in Production Example 1.
[Fig. 3] Fig 3 is a graph showing a $^{13}C$-NMR spectrum of 4-(3-propoxybutyl)phenol in Production Example 1.
[Fig. 4] Fig 4 is a graph showing a $^{13}C$-NMR spectrum of 4-(3-isopropoxybutyl)phenol in Production Example 1.
[Fig. 5] Fig 5 is a graph showing a $^{13}C$-NMR spectrum of 4-(3-octyloxybutyl)phenol in Production Example 1.

[Embodiment for carrying out the invention]

Hereinafter, embodiments of the present invention will be described in detail.
4-(3-alkoxybutyl)phenol (compound (1)) used in the present invention is a compound represented by the above-mentioned formula (1). The compound (1) is described in, for example, Patent Document 2 and is already known. However, the compound (1) has been known to be useful as organic electronic materials such as a liquid crystal compound as in Patent Document 2, but it has not been known at all whether or not the compound (1) can be applied to the skin. In the formula (1), R represents an alkyl group having 1 to 8 carbon atoms, and may be linear or branched, and may have a substituent such as an amino group and a hydroxyl group. Specifically, examples of the alkyl group having 1 to 8 carbon atoms include a methyl group, an ethyl group, a propyl group, an isopropyl group, a butyl group, a hexyl group, an octyl group, a 2-ethyl hexyl group, and the like. Furthermore, examples of groups having an amino group and a hydroxyl group include groups derived from 1,3-propanediol, propylene glycol, glycerine, serinol, 1,3-butylene glycol, and the like. Among them, an alkyl group having 1 to 6 carbon atoms is preferable, an alkyl group having 1 to 4 carbon atoms is more preferable, a methyl group, an ethyl group, a n-propyl group, and an isopropyl group are further preferable,

and an ethyl group is particularly preferable. The groups in this range provide a better melanin production-inhibitory effect and solubility in various solvents. However, R does not include saccharides.

The compound (1) to be used in the present invention can be manufactured by a known organic synthesis reaction using as a starting material, 4-(4-hydroxyphenyl)-2-butanone obtained by a known synthetic method or a material separated and purified from an extract of a plant such as raspberry containing 4-(4-hydroxyphenyl)-2-butanone. For example, the manufacturing method includes a method of reacting 4-(4-hydroxyphenyl)-2-butanone with arbitrary alcohol and triethyl silane in a trifluoroacetic acid solvent. Furthermore, the compound (1) can be obtained by treating an extract of a plant such as raspberry by an appropriate method, followed by separating and purifying the target 4-(3-alkoxybutyl) phenol compound.

The compound (1) obtained by the above-mentioned method and the like has optical isomers, and a (+) isomer or a (-) isomer may be used singly or a combination thereof may be used.

Furthermore, as the compound (1) to be used in the present invention, any one type among 4-(3-alkoxybutyl) phenol represented by the above-mentioned formula (1) may be used, or a mixture of two or more thereof may be used.

As shown in the below-mentioned Examples, the compound (1) has better melanin production-inhibitory and whitening actions than a compound described in Patent Document 1. Furthermore, since the compound (1) has high solubility in a solvent such as ester oil and alcohol, and does not have an unstable bond such as an ester bond, it can stably and safely be blended in a composition for external use such as cosmetics.

The composition for external use on skin of the present invention can generally be contained in a content of 0.0001 to 20% by mass, preferably 0.01 to 10% by mass, and more preferably 0.1 to 5% by mass based on the total amount of the composition for external use on skin, from the viewpoint of whitening action and safety although the content is not indiscriminately limited and varies depending upon the purpose or targets for blending the compound (1).

The composition for external use on skin of the present invention is preferably a melanin production inhibitor and a whitening cosmetic based on the melanin production-inhibitory action and the whitening action of compound (1). The composition for external use on skin of the present invention can be used in appropriate combination with, in addition to the compound (1), whitening agents such as known hydroquinone, arbutin, ellagic acid, vitamin C and derivatives thereof (for example, ascorbic acid glucoside, sodium ascorbyl phosphate, disodium ascorbyl sulfate, and magnesium ascorbyl phosphate), biphenyl derivatives (for example, dehydrodicreosol, and 2,2'-dihydroxy-5,5'-dipropylbiphenyl), and 4-(4-hydroxyphenyl)-2-butanone described in Patent Document 1 and derivatives thereof, melanin production inhibitors such as 4-(4-hydroxyphenyl)-2-butanol, 4-(3-hydroxybutyl)phenyl acetate, firethorn(Pyracantha fortuneana) extract, Dioscorea composita extract, bergenia extract, camomile extract, adenosine 5'-1-phosphoric acid and salts thereof, linoleic acid derivatives, vitamin B3 and derivatives thereof, tranexamic acid and salts of tranexamic acid, and tranexamic acid derivatives.

The composition for external use on skin of the present invention can appropriately contain, in addition to the above, moisturizing agents such as hyaluronic acid, polyol, and sugar alcohol, coloring pigments such as a tar-based coloring agents and iron oxide, preservatives such as paraben, anionic surfactants such as fatty acid soap, and sodium cetyl sulfate, nonionic surfactants such as polyoxyethylene alkyl ether, polyoxyethylene fatty acid ester, polyoxyethylene polyol fatty acid ester, polyoxyethylene hydrogenated castor oil, polyol fatty acid ester, and polyglycerin fatty acid ester, cationic surfactants such as tetraalkyl ammonium salt, amphoteric surfactants such as betaine type, sulfobetaine type, sulfo-amino acid type, and sodium N-stearoyl-L-glutamate, natural surfactants such as lecithin and lysophosphatidylcholine, natural polymers such as gelatin, casein, starch, gum arabic, karaya gum, guar gum, locust bean gum, tragacanth gum, quince seed, pectin, carageenan, and sodium alginate, semisynthetic polymer such as methylcellulose, hydroxyethylcellulose, hydroxypropylcellulose, sodium carboxymethylcellulose and ethylcellulose, synthetic polymers such as polyvinyl alcohol, polyvinyl methyl ether and copolymer, polyvinylpyrrolidone, sodium polyacrylate, carboxyvinyl polymer, and polyethylene oxide polymer, thickeners such as xanthene, pigments such as titanium oxide, antioxidants such as dibutylhydroxytoluene, and the like, within a range that does not damage the objects of the present invention.

The form of the composition for external use on skin of the present invention may generally be used as a bath salt, in addition to a skin cosmetic to be applied to the skin. In particular, the present invention is preferably applied to whitening cosmetics because the 4-(3-alkoxybutyl)phenol compound has an excellent melanin production-inhibitory action. The formulation forms are not particularly limited as long as the 4-(3-alkoxybutyl)phenol compound of the present invention can stably be blended, and examples thereof include liquids such as lotion, emulsions such as gel, emulsified lotion, and cream, sheets, sticks, and granules or powders using an appropriate excipient and the like. As the emulsion, any of a water-in-oil type emulsion, an oil-in-water type emulsion, and multi-emulsion may be employed.

[Examples]

Hereinafter, the present invention will be described in detail based on the Production Examples, the Test Examples, and the Examples, but the present invention is not particularly limited thereto. Note here that as 4-(4-hydroxyphenyl)-2-butanone mentioned below, a generally available reagent was used. Furthermore, an NMR spectrum was

measured by using $CDCl_3$ as a measurement solvent, and JEOL JNM-LA400 manufactured by JEOL Ltd.

Production Example 1: Synthesis of a 4-(3-alkoxybutyl)phenol compound

Synthesis of a 4-(3-alkoxybutyl)phenol compound was carried out according to the method described by Michael et al. (Journal of the American Chemical Society, pp.3659-3661, 1972). 4-(4-hydroxyphenyl)-2-butanone (10 mmol) and triethylsilane (11 mmol) were dissolved in various alcohols (125 mmol), to which trifluoroacetic acid (375 mmol) was added dropwise under ice cooling. The temperature was returned to room temperature and the resulting mixture was stirred for 24 hours. Iced water (100 mL) was added to the reaction solution, and the mixture was extracted with hexane (100 mL) twice. The obtained hexane layer was washed several times with a saturated saline solution and a saturated sodium hydrogencarbonate solution, and dried with anhydrous magnesium sulfate and filtered. The filtrate was concentrated under reduced pressure, purified by silica gel column chromatography (hexane / ethyl acetate solvent) to obtain the below-mentioned compounds. The $^{13}$C-NMR spectra of these compounds are shown in Figs. 1 to 5.

4-(3-methoxybutyl)phenol: colorless oily product, $^{1}$H-NMR ($CDCl_3$) δ: 7.03 (2H, d, J=8.3 Hz), 6.75 (2H, d, J=8.3 Hz), 3.35 (3H, s), 3.33 (1H, m), 2.60 (2H, m), 1.83 (1H, m), 1.67 (1H, m), 1.18 (3H, d, J=6.2 Hz). $^{13}$C-NMR ($CDCl_3$) δ: 154.0, 133.8, 129.4, 115.2, 76.4, 55.8, 38.1, 30.7, 18.9. (see Fig. 1)

4-(3-ethoxybutyl)phenol: colorless oily product, $^{1}$H-NMR ($CDCl_3$) δ: 7.05 (2H, d, J=8.4 Hz), 6.75 (2H, d, J=8.4 Hz), 3.57 (1H, m), 3.40 (2H, m), 2.61 (2H, m), 1.80 (1H, m), 1.66 (1H, m), 1.21 (3H, t, J=6.8 Hz), 1.16 (3H, d, J=6.2 Hz). $^{13}$C-NMR ($CDCl_3$) δ: 153.8, 134.0, 129.4, 115.2, 74.7, 63.7, 38.5, 30.9, 19.7, 15.5. (see Fig. 2)

4-(3-propoxybutyl)phenol : colorless oily product, $^{1}$H-NMR ($CDCl_3$) δ: 7.04 (2H, d, J=8.8 Hz), 6.73 (2H, d, J=8.4 Hz), 3.45 (1H, m), 3.37 (1H, m), 3.29 (1H, m), 2.61 (2H, m), 1.82 (1H, m), 1.66 (1H, m), 1.58 (2H, q, J=7.3 Hz), 1.15 (3H, d, J=6.0 Hz), 0.93 (3H, t, J=7.3 Hz). $^{13}$C-NMR ($CDCl_3$) δ: 153.5, 134.5, 129.4, 115.1, 74.6, 70.2, 38.7, 30.9, 23.3, 19.7, 10.8. (see Fig. 3)

4-(3-isopropoxybutyl)phenol: colorless oily product, $^{1}$H-NMR ($CDCl_3$) δ: 7.04 (2H, d, J=8.8 Hz), 6.74 (2H, d, J=8.8 Hz), 3.64 (1H, septet, J=6.2 Hz), 3.46 (1H, m), 2.65 (1H, m), 2.53 (1H, m), 1.76 (1H, m), 1.65 (1H, m), 1.15 (3H, d, J=6.0 Hz), 1.14 (3H, d, J=6.4 Hz, 1.13 (3H, d, J=6.0 Hz). $^{13}$C-NMR ($CDCl_3$) δ: 153.5, 134.5, 129.4, 115.1, 72.3, 69.3, 39.1, 31.1, 23.3, 22.6, 20.8. (see Fig. 4)

4-(3-octyloxybutyl)phenol: colorless oily product, $^{1}$H-NMR ($CDCl_3$) δ: 7.02 (2H, d, J=8.0 Hz), 6.75 (2H, d, J=8.0 Hz), 3.53 (1H, m), 3.37 (2H, m), 2.61 (2H, m), 1.84 (1H, m), 1.67 (1H, m), 1.58 (2H, m), 1.26 (m), 1.18 (3H, d, J=6.0 Hz), 0.86 (3H, t, J=6.6 Hz). $^{13}$C-NMR ($CDCl_3$) δ: 153.8, 134.1, 129.4, 115.2, 74.8, 68.6, 38.5, 31.8, 30.9, 30.0, 29.4, 29.2, 26.2, 22.6, 19.6, 14.1. (see Fig. 5)

Test Example 1: Solubility Test

By using 4-(3-ethoxybutyl)phenol obtained in the above-mentioned Production Example 1, the following solubility test was carried out. Note here that as a comparison target, 4-(3-hydroxybuty)phenol was used.

(Test method)

To 0.1 g of each compound, 1 mL of each solvent described in the following table 1 was added. The mixture was heated at 40°C for 10 min., and then the temperature was returned to room temperature. The solubility in the solvent at this time was evaluated by visual observation. The evaluation was carried out in which a case where it was dissolved was evaluated as "a" and a case where insolubles were left was evaluated as "b."

The results are shown in Table 1.

[Table 1]

| | 4- (3-Ethoxybutyl)phenol | 4-(3-Hydroxybutyl)phenol |
|---|---|---|
| Octyldodecyl myristate | a | a |
| Glyceryl tri(2-ethylhexanoate) | a | b |
| Isocetyl myristate | a | b |
| Methylphenyl polysiloxane (note 1) | a | b |
| Dipropylene glycol | a | a |

(continued)

| | 4- (3-Ethoxybutyl)phenol | 4-(3-Hydroxybutyl)phenol |
|---|---|---|
| Ethanol | a | a |
| (Note 1) Silicon FZ209 (manufactured by Dow Corning Toray Co., LTD.) | | |

From Table 1, it is shown that the 4-(3-alkoxybutyl)phenol compound of the present invention has better solubility in a low polarity solvent than 4-(3-hydroxybutyl)phenol.

Test Example 2: in vitro melanin production inhibition test

The 4-(3-alkoxybutyl)phenol compound obtained in the above-mentioned Production Example 1 was subjected to the following melanin production inhibition test. Note here that as a comparison target, arbutin, ascorbic acid, kojic acid, and linoleic acid, which are known to have a melanin production-inhibitory action, were used.

(Test method)

B16 melanoma cells were seeded in a minimum essential medium (MEM) supplemented with 10 vol% fetal bovine serum in 12-well plates at $1 \times 10^4$ cells/well, and pre-cultured for 24 hours by a usual method. After pre-culture, the medium was replaced to a test medium to which each evaluation sample had been added at various concentrations, and the medium was cultured for 72 hours. As the test medium, the above-mentioned pre-culture medium to which theophylline had been added at 2 mmol/L was used. After culture, cells were collected and dissolved in 1 mol/L of an aqueous solution of sodium hydroxide containing 10 vol% dimethyl sulfoxide, and an OD475 value of the solution was measured and the value was defined as an index of the amount of melanin. Furthermore, at the same time, by using Coomasie Plus Protein Assay Kit (manufactured by PIERCE), the total amount of protein in the solution was measured, and the amount of melanin per unit amount of protein was calculated.

($IC_{50}$)

Based on the amount of melanin per unit amount of protein, the melanin production inhibition rate was calculated per each addition concentration from the following formula.

$$\text{Melanin production inhibition rate (\%)} = (A-B)/A \times 100$$

(wherein A denotes the amount of melanin per unit amount of protein when no sample was added, and B denotes the amount of melanin per unit amount of protein when a sample was added).
Next, a graph in which the concentration of the added sample is plotted on the axis of abscissas, and a melanin production inhibition rate is plotted in the axis of ordinates was formed, and concentration at which each evaluation sample inhibits the production of melanin by 50% ($IC_{50}$) was determined from this graph.

The results are shown in Table 2.

[Table 2]

| Compound | $IC_{50}$ (μg/mL) |
|---|---|
| 4-(3-Methoxybutyl)phenol | 2.0 |
| 4-(3-Ethoxybutyl)phenol | 2.0 |
| 4-(3-Propoxybutyl)phenol | 2.5 |
| 4-(3-Isopropoxybutyl)phenol | 2.0 |
| Arbutin | $3.0\times10^1$ |
| Ascorbic acid | $6.0\times10^1$ |
| Kojic acid | $1.8\times10^2$ |
| Linoleic acid | 4.0 |

From Table 2, it is shown that the 4-(3-alkoxybutyl)phenol compound of the present invention exhibits a better melanin production-inhibitory effect than arbutin, ascorbic acid, kojic acid, and linoleic acid as known melanin production inhibitors.

Test Example 3: Melanin production inhibition Test Using Three-Dimensional Cultured Skin Model

4-(3-alkoxybutyl)phenol obtained in the above-mentioned Production Example 1 was subjected to the below-mentioned melanin production inhibition test. Note here that as a comparison target, 4-(3-hydroxybutyl)phenol in which an alkyl group had been removed from 4-(3-alkoxybutyl)phenol -and 4-(3-acetoxybutyl)phenol and 4-(3-propanoyloxybutyl)phenol as an ester compound having the same number of carbon atoms, were used.

(Test Method)

To a skin model cup of a cultured skin model containing melanin cells (MEL-300, Asian Doner, manufactured by Kurabo Industries, Ltd.), 0.2 mL of a solution of a test substance was placed, and the skin model was cultured for 14 days by using an LLMM medium attached to the product. During the culture period, the medium was replaced by a new medium every three days. After culture for 14 days, the skin model was washed, the survival rate was measured by fluorescence measurement (Excitation: 560 nm, Emission: 590 nm) using an AlamarBlue reagent (manufactured by Molecular Probe), and the amount of melanin synthesized in each skin model was measured. The amount of melanin synthesized was determined by the colorimetric determination (405 nm) of a solution obtained by immersing each skin model in PBS so as to separate a cell layer, then washing it with PBS and hydrous ethanol, adding 0.2 mL of a 2N NaOH aqueous solution, and extracting melanin at 100°C for three hours.

(Test Result)

The results are shown in Table 3.
[Table 3]

| Compound | Addition concentration (μM) | Melanin production inhibition rate (% with respect to control) |
|---|---|---|
| 4-(3-Methoxybutyl)phenol | 100 | 55.6% |
| 4-(3-Ethoxybutyl)phenol | 100 | 56.0% |
| 4-(3-Propoxybutyl)phenol | 50 | 69.5% |
| 4-(3-Isopropoxybutyl)phenol | 50 | 56.9% |
| 4-(3-Hydroxybutyl)phenol | 100 | 18.6% |
| 4-(3-Acetoxybutyl)phenol | 100 | 26.7% |
| 4-(3-Propanoyloxybutyl)phenol | 50 | 18.4% |

From the result of Table 3, it is shown that the 4-(3-alkoxybutyl)phenol compound of the present invention has a better whitening action than Comparative Examples. In particular, from the comparison between 4-(3-ethoxybutyl) phenol and 4-(3-acetoxybutyl)-phenol, as well as the comparison between 4-(3-propoxybutyl)phenol and 4-(3-propanoyloxy butyl)phenol which have the same number of carbon atoms as each other, the compound (1) having an ether bond better contributes to the whitening action than the compound having an ester bond. Note here that in all of the above-mentioned test concentrations, no cytotoxicity was detected.

Test Example 3: Whitening Practical Test

By using skin cream (Example 1) in which the 4-(3-ethoxybutyl)phenol compound obtained in the above-mentioned Production Example 1 was blended, the below-mentioned whitening practical test was carried out. As a comparison target, skin cream (Comparative Example 1) in which arbutin that is known to have a whitening action was mixed was used.

(Test Method)

The skin of the forearm flexor side of each of 20 subjects was exposed to the sun light of summer season for three hours (1.5 hours per day, for two continuous days). After exposure, the skin cream of the Example 1 of the below-mentioned Table 4 was applied to the skin of the left forearm flexor side of the subject once every morning and evening for 13 continuous weeks. Furthermore, the skin cream of the Comparative Example 1 of the below-mentioned Table 3 was applied to the skin of the right forearm flexor side of the subject in the same conditions.
After the final application was finished, the degree of whitening of the skin of the right and left forearm flexor sides, before and after applying the skin cream, were evaluated by special judge persons. In evaluation, subjects in which the whitening effect had been determined was evaluated as "whitening effect was observed" and the number of the subjects was shown.
[Table 4]

| (Name of Component) | (Amount blended: % by mass) | |
|---|---|---|
| | Examples 1 | Comparative Example 1 |
| Cetanol | 5 | 5 |
| Lipophilic glyceryl monostearate | 1 | 1 |
| Polyoxyethylene cetyl ether (2E.O.) | 0. 1 | 0. 1 |
| Methylphenyl polysiloxane | 2 | 2 |
| Squalane | 2 | 2 |
| Sucrose fatty acid ester | 0. 5 | 0. 5 |
| 4-(3-Ethoxybutyl)phenol | 2 | - |
| Arbutin | - | 3 |
| Alkyl acrylate copolymer | 0. 1 | 0. 1 |
| Purified water | Balance | Balance |

(Results)

The results are shown in Table 5. From the results, it is shown that the skin cream in which the 4-(3-ethoxybutyl) phenol compound of Production Example 1 of the present invention is blended is much better as a whitening cosmetic than the skin cream of Comparative Example 1 in which arbutin had been blended. Note here that during the test period, no subjects experienced skin irritation reaction and skin sensitization reaction in a site to which the skin cream of Example 1 had been applied. Thus, it was confirmed that the embodiment of the invention was also safe in the form of a formulation.
[Table 5]

| | Whitening Effect was Observed (persons) |
|---|---|
| **Example 1** | **15** |
| Comparative Example 1 | **8** |

Next, by using each compound of Production Example 1, each cosmetic was manufactured by a usual method according to the below-mentioned components to be blended.

Example 2 (Skin lotion)

[Table 6]

| Components of raw material | Amount blended (% by mass) |
|---|---|
| Ethanol | 10. 0 |
| POE (20) sorbitan monolaurate | 5. 0 |
| Phenoxyethanol | 0. 1 |

(continued)

| Components of raw material | Amount blended (% by mass) |
|---|---|
| Perfume | 0. 05 |
| 4-(3-Methoxybutyl)phenol | 2. 0 |
| Glycerin | 5. 0 |
| Xanthan gum | 0. 1 |
| Hydroxyethylcellulose | 0. 1 |
| Purified water | Balance |

Example 3 (Skin cream)

[Table 7]

| Components of raw material | Amount blended (% by mass) |
|---|---|
| Glycerin monostearate | 2. 0 |
| Beeswax | 1. 0 |
| POE (20) sorbitan monooleate | 6. 0 |
| Vaseline | 4. 0 |
| Liquid paraffin | 12. 0 |
| 4-(3-Methoxybutyl)phenol | 2. 0 |
| N-Stearoyl-L-sodium glutamate | 1.0 |
| Carageenan | 0. 3 |
| Methylparaben | 0. 1 |
| Purified water | Balance |

Example 4 (Cream)

[Table 8]

| Components of raw material | Amount blended(% by mass) |
|---|---|
| Stearic acid | 1. 0 |
| Glycerin monoisostearate | 2. 0 |
| Behenyl alcohol | 2. 0 |
| White beeswax | 1. 0 |
| Isocetyl myristate | 1. 0 |
| Sorbitan isostearate | 1. 0 |
| Retinyl palmitate | 0. 1 |
| Hydrogenated lecithin | 0. 1 |
| Ubidecarenone | 0. 03 |
| Phytosterol | 0. 1 |
| Vegetable squalane | 5. 0 |

(continued)

| Components of raw material | Amount blended(% by mass) |
|---|---|
| Hydrogenated polydecene | 5. 0 |
| Dicapryl carbonate | 5. 0 |
| 1,3-Butylene glycol | 5. 0 |
| Concentrated glycerin | 5. 0 |
| N-Acetylglucosamine | 0. 1 |
| Disodium ascorbic acid sulfate | 0. 2 |
| $\gamma$-Aminobutyric acid | 0. 1 |
| Sodium N-stearoylglutamate | 0. 2 |
| Monoammonium glycyrrhizinate | 0. 1 |
| Edelweiss extract (Note 2) | 0. 2 |
| Yeast extract (Note 3) | 0. 2 |
| Acrylic acid - alkyl methacrylate copolymer (Note 4) | 0. 05 |
| Niacinamide | 0. 1 |
| Creatine | 0. 2 |
| Carnitine chloride | 0. 1 |
| Firethorn extract (Note 5) | 0. 1 |
| 4-(3-Ethoxybutyl)phenol | 3. 0 |
| Purified water | Balance |
| (Note 2) Edelweiss extract GC (manufactured by Pentapharm Ltd.)<br>(Note 3) Dismucin BTJ (manufactured by Pentapharm Ltd.)<br>(Note 4) PEMULEN TR-1 (manufactured by Lubrizol Advanced Materials)<br>(Note 5) Firethorn extract liquid (manufactured by Suntory Holdings Limited) | |

Example 5 (Cream)

[Table 9]

| Components of raw material | Amount blended (%by mass) |
|---|---|
| Isostearic acid | 1. 0 |
| Glycerin monoisostearate | 2. 0 |
| Behenyl alcohol | 2. 0 |
| White beeswax | 1. 0 |
| Isocetyl myristate | 1. 0 |
| Sorbitan isostearate | 1. 0 |
| Retinyl palmitate | 0. 1 |
| Hydrogenated lecithin | 0. 1 |
| Ubidecarenone | 0. 03 |
| Phytosterol | 0. 1 |
| Vegetable squalane | 5. 0 |

(continued)

| Components of raw material | Amount blended (%by mass) |
|---|---|
| Dicapryl carbonate | 5. 0 |
| Methyl parahydroxybenzoate | 0. 2 |
| 1,3-Butylene glycol | 10. 0 |
| Concentrated glycerin | 5. 0 |
| N-Acetylglucosamine | 0. 1 |
| Disodium ascorbic acid sulfate | 0. 2 |
| γ-Aminobutyric acid | 0. 1 |
| Sodium N-stearoylglutamate | 0. 2 |
| Monoammonium glycyrrhizinate | 0. 1 |
| Edelweiss extract (Note 2) | 0. 2 |
| Yeast extract (Note 3) | 0. 2 |
| Acrylic acid - alkyl methacrylate copolymer | 0. 05 |
| Niacinamide | 0. 1 |
| Creatine | 0. 2 |
| Carnitine chloride | 0. 1 |
| Magnesium ascorbyl phosphate | 0. 1 |
| Dioscorea Composita extract (Note 6) | 0. 1 |
| Sambucus extract (Note 7) | 0. 1 |
| 4-(3-Methoxybutyl)phenol | 5. 0 |
| Purified water | Balance |
| (Note 6) Dioscorea Composita Root Extract (manufactured by Mitsui-Chemicals Inc.)<br>(Note 7) Sambucus extract BG (manufactured by MARUZEN PHARMACEUTICALS Co., LTD.) | |

Examples 6 to 8 (Sunscreen)

[Table 10]

| Components of raw material | Amount blended (% by mass) | | |
|---|---|---|---|
| | Example 6 | Example 7 | Example 8 |
| Dioctyl ether | 22. 0 | 15.0 | 10. 0 |
| Comodified silicone (Note 8) | 2. 0 | 2. 0 | 2. 0 |
| Glyceryl tris(2-ethyl hexanoate) | - | - | 0.5 |
| Hydrogenated oil | - | - | 0. 1 |
| Methylphenyl polysiloxane | - | 3. 0 | - |
| Phytosteryl macadamia nut fatty acid | - | - | 2. 0 |
| Titanium oxide | 5. 0 | - | 4. 0 |
| Zinc oxide | 5. 0 | - | 4. 0 |

(continued)

| Components of raw material | Amount blended (% by mass) | | |
|---|---|---|---|
| | Example 6 | Example 7 | Example 8 |
| Ethyl hexyl methoxycinnamate | 10. 0 | 10. 0 | 10. 0 |
| 4-(3-Octyloxybutyl)phenol | 1. 0 | 1. 0 | 0. 5 |
| Phenoxyethanol | 0. 3 | 0. 3 | 0. 3 |
| Magnesium chloride | 1. 0 | 1. 0 | 1. 0 |
| 1,3-Butylene glycol | 5. 0 | 5. 0 | 5. 0 |
| Royal jelly extract (Note 9) | 1. 0 | 1. 0 | 1. 0 |
| Aloe extract product (Note 10) | 0. 1 | 0. 1 | 0. 1 |
| Phellodendri cortex extract (Note 11) | 0. 5 | 0. 5 | 0. 5 |
| Yeast extract (Note 3) | 1. 0 | 1. 0 | 1. 0 |
| Purified water | Balance | Balance | Balance |
| (Note 8) ABIL EM90 (manufactured by Gold Schmidt GmbH)<br>(Note 9) Royal jelly (manufactured by API Co., Ltd)<br>(Note 10) Aloe extracted gel BG (manufactured by MARUZEN PHARMACEUTICALS Co., LTD.)<br>(Note 11) Phellodendri cortex extracted liquid J (manufactured by MARUZEN PHARMACEUTICALS Co., LTD.) | | | |

Examples 9 to 11 (Essence)

[Table 11]

| Components of raw material | Amount blended (% by mass) | | |
|---|---|---|---|
| | Example 9 | Example 10 | Example 11 |
| Comodified silicone (Note 8) | 2. 0 | 2. 0 | 2. 0 |
| POE modified silicone dispersion solution (Note 12) | - | 2. 0 | - |
| Squalane | - | - | 10. 0 |
| Decamethyl cyclopentasiloxane | 15. 0 | 20. 0 | 10. 0 |
| Methyl polysiloxane (100 cs) | 5.0 | 2. 0 | 3. 0 |
| Branched long-chain fatty acid cholesteryl ester (Note 13) | - | - | 2. 0 |
| Silicone elastomer dispersion solution (Note 14) | 5. 0 | 2. 0 | - |
| 4-(3-Mothoxybutyl)phenol | 0. 01 | 0. 1 | 0. 5 |
| Methyl parahydroxybenzoate | 0. 05 | 0. 05 | 0. 05 |
| Sodium chloride | 1. 0 | 1. 0 | 1. 0 |
| Dipropylene glycol | 5. 0 | 5. 0 | 5. 0 |
| Concentrated glycerin | 5. 0 | 5. 0 | 5. 0 |
| Raffinose | 1. 0 | 1. 0 | 1. 0 |
| Mixed isomerized sugar (Note 15) | 1. 0 | 1. 0 | 1. 0 |
| Glycyrrhiza extract (Note 16) | 0. 1 | 0. 1 | 0. 1 |
| N-Methyl-L-serine | 0. 5 | 0. 5 | 0. 5 |

(continued)

| Components of raw material | Amount blended (% by mass) | | |
|---|---|---|---|
| | Example 9 | Example 10 | Example 11 |
| Purified water | Balance | Balance | Balance |

(Note 12) Silicone BY22-008 (manufactured by Dow Corning Toray Co., LTD.)
(Note 13) YOFCO CLE-NH (manufactured by Nippon Fine Chemical)
(Note 14) TORAYFIL (manufactured by Dow Corning Toray Silicon Co., LTD.)
(Note 15) PENTAVITIN (manufactured by Pentapharm Ltd.)
(Note 16) Glycyrrhiza extracted liquid (manufactured by MARUZEN PHARMACEUTICALS Co., LTD.)

Examples 12 to 13 (Skin lotion)

[Table 12]

| Components of raw material | Amount blended (% by mass) | |
|---|---|---|
| | Example 12 | Example 13 |
| 4-(3-methoxybutyl)phenol | 0. 1 | 0. 01 |
| Phenoxyethanol | 0. 2 | 0. 2 |
| 1,3-Butylene glycol | 0. 3 | 0. 3 |
| Dipropylene glycol | - | 5. 0 |
| Raffinose | 5. 0 | 5. 0 |
| Dimethyl hyaluronate silanol solution (Note 17) | 0. 1 | 0. 1 |
| MPC polymer (Note 18) | 0. 1 | 0. 1 |
| Ethanol | - | 1. 0 |
| Pectin | - | 0.05 |
| Xanthan gum | - | 0.01 |
| Sodium citrate | 0. 05 | 0. 05 |
| Equisetum arvense extract (Note 19) | 0. 1 | 0. 1 |
| Diisopropylamine dichloroacetate | 0. 2 | 0. 2 |
| dl-$\alpha$-tocopherol acetate | 0. 1 | 0. 1 |
| $\gamma$-Amino-$\beta$-hydroxybutyric acid | 0. 2 | 0. 2 |
| Sodium hyaluronate | 0. 001 | 0. 001 |
| Dipotassium glycyrrhizinate | 0. 2 | 0. 2 |
| Pentapeptide-3 (Note 20) | 0. 05 | 0. 05 |
| Decarboxy carnosine hydrochloride | 0. 05 | 0. 05 |
| Perfume | 0. 02 | 0. 02 |
| Purified water | Balance | Balance |

(Note 17) DSHC-N (manufactured by EXYMOL)
(Note 18) LIPIDURE PMB (manufactured by NOF Coporation)
(Note 19) Equisetum arvense extract (manufactured by MARUZEN PHARMACEUTICALS Co., LTD.)
(Note 20) MATRIXYL (manufactured by Croda Japan KK)

Example 14 (Emulsion)

[Table 13]

| Components of raw material | Amount blended (% by mass) |
|---|---|
| Methylphenyl polysiloxane (Note 21) | 3. 0 |
| Dicapryl carbonate | 1.0 |
| Olive oil | 1. 0 |
| Meadowfoam oil | 0. 1 |
| POE(20) Sorbitan monolaurate | 0. 5 |
| dl-α-tocopherol nicotinate | 0. 01 |
| POE(60) Hydrogenated castor oil | 2. 0 |
| Shea butter (Note 22) | 0. 01 |
| Ascorbyl tetraisopalmitate | 0. 1 |
| N-Acetylglucosamine | 0.02 |
| Yeast extract (Note 3) | 3. 0 |
| 1,3-Butylene glycol | 3. 0 |
| Sorbitol solution | 3. 0 |
| Polyethylene glycol 1000 | 1.0 |
| Carboxyvinyl polymer | 0. 1 |
| 4-(3-Octyloxybutyl)phenol | 0. 01 |
| Methyl parahydroxybenzoate | 0. 2 |
| Tremella fuciformis polysaccharide (Note 23) | 0. 05 |
| Edetate sodium | 0. 02 |
| Potassium hydroxide | 0. 05 |
| Xanthan gum | 0. 05 |
| Polyacrylamide (Note 24) | 0. 01 |
| Purified water | Balance |
| (Note 21) Cosmeserve WP-58MP (manufactured by Dainihon Kasei Co., Ltd.)<br>(Note 22) Cropure SB (manufactured by Croda Japan KK)<br>(Note 23) Tremoist-TP (manufactured by NIPPON FINE CHEMICAL CO., LTD.)<br>(Note 24) Cosmedia SP (manufactured by Cognis) | |

The 4-(3-alkoxybutyl)phenol compound of the present invention can be applied to cosmetic formulations of Examples 2 to 14. When they are used, an excellent melanin production-inhibitory effect and whitening effect were observed.

[Industrial Applicability]

A 4-(3-alkoxybutyl)phenol compound of the present invention is excellent in stability, and can be applied in wide range of formulations, for example, a composition for external use on skin such as lotion, emulsion, cream, packs, and bath salt. Furthermore, a 4-(3-alkoxybutyl)phenol compound of the present invention has excellent melanin production-inhibitory and whitening effects, and therefore it is very useful in the field of skin beauty treatment.

## Claims

**1.** A composition for external use on skin comprising a compound represented by the following formula (1):

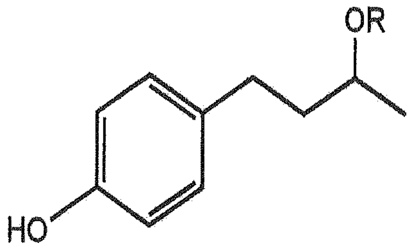

(1)

wherein R represents an alkyl group having 1 to 8 carbon atoms.

**2.** The composition for external use on skin according to claim 1, wherein R in the formula (1) is an alkyl group having 1 to 4 carbon atoms.

**3.** The composition for external use on skin according to claim 1, wherein R in the formula (1) is a methyl group, an ethyl group, a n-propyl group, or an isopropyl group.

**4.** The composition for external use on skin according to claim 1, wherein R in the formula (1) is an ethyl group.

**5.** A whitening cosmetic comprising a compound represented by the following formula (1):

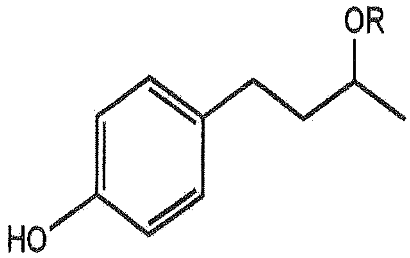

(1)

wherein R represents an alkyl group having 1 to 8 carbon atoms.

**6.** The whitening cosmetic according to claim 5, wherein R in the formula (1) is an alkyl group having 1 to 4 carbon atoms.

**7.** The whitening cosmetic according to claim 5, wherein R in the formula (1) is a methyl group, an ethyl group, a n-propyl group, or an isopropyl group.

**8.** The whitening cosmetic according to claim 5, wherein R in the formula (1) is an ethyl group.

**9.** A melanin production inhibitor comprising a compound represented by the following formula (1) as an active ingredient:

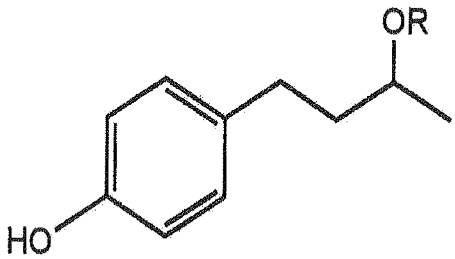

(1)

wherein R represents an alkyl group having 1 to 8 carbon atoms.

**10.** The melanin production inhibitor according to claim 9, wherein R in the formula (1) is an alkyl group having 1 to 4 carbon atoms.

**11.** The melanin production inhibitor according to claim 9, wherein R in the formula (1) is a methyl group, an ethyl group, a n-propyl group, or an isopropyl group.

**12.** The melanin production inhibitor according to claim 9, wherein R in the formula (1) is an ethyl group.

**13.** A compound represented by the following formula (1) for external use on skin:

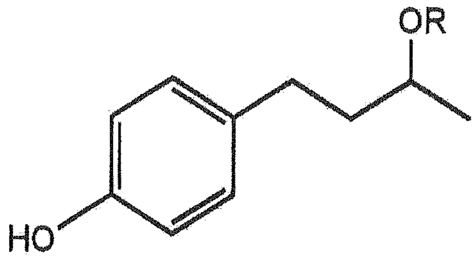

(1)

wherein R represents an alkyl group having 1 to 8 carbon atoms.

**14.** The compound according to claim 13, wherein R in the formula (1) is an alkyl group having 1 to 4 carbon atoms.

**15.** The compound according to claim 13, wherein R in the formula (1) is a methyl group, an ethyl group, a n-propyl group, or an isopropyl group.

**16.** The compound according to claim 13, wherein R in the formula (1) is an ethyl group.

**17.** The compound according to any one of claims 13 to 16, wherein the external use on skin is application to the skin for whitening.

**18.** The compound according to any one of claims 13 to 16, wherein the external use on skin is application to the skin for inhibiting production of melanin in the skin.

**19.** Use of a compound represented by the following formula (1) for manufacturing a composition for external use on skin:

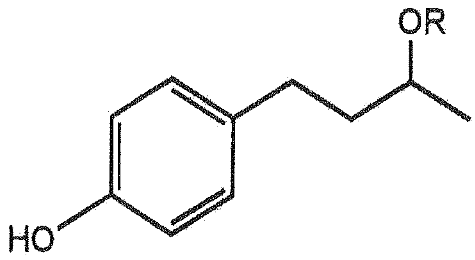

(1)

wherein R represents an alkyl group having 1 to 8 carbon atoms.

**20.** The use according to claim 19, wherein R in the formula (1) is an alkyl group having 1 to 4 carbon atoms.

**21.** The use according to claim 20, wherein R in the formula (1) is a methyl group, an ethyl group, a n-propyl group, or an isopropyl group.

**22.** The use according to claim 20, wherein R in the formula (1) is an ethyl group.

**23.** Use of a compound represented by the following formula (1) for manufacturing a whitening cosmetic:

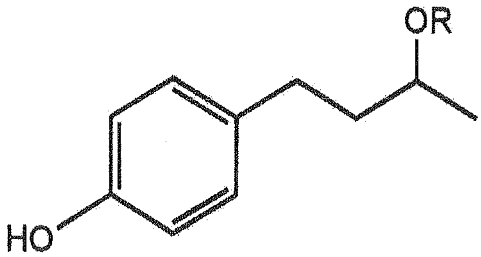

(1)

wherein R represents an alkyl group having 1 to 8 carbon atoms.

**24.** The use according to claim 23, wherein R in the formula (1) is an alkyl group having 1 to 4 carbon atoms.

**25.** The use according to claim 23, wherein R in the formula (1) is a methyl group, an ethyl group, a n-propyl group, or an isopropyl group.

**26.** The use according to claim 23, wherein R in the formula (1) is an ethyl group.

**27.** Use of a compound represented by the following formula (1) for manufacturing a melanin production inhibitor:

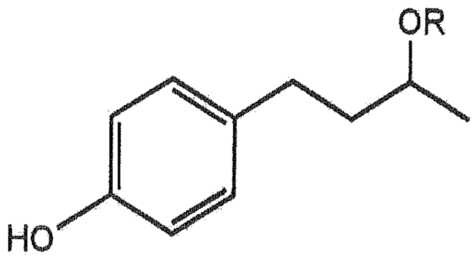

(1)

wherein R represents an alkyl group having 1 to 8 carbon atoms.

**28.** The use according to claim 27, wherein R in the formula (1) is an alkyl group having 1 to 4 carbon atoms.

**29.** The use according to claim 27, wherein R in the formula (1) is a methyl group, an ethyl group, a n-propyl group, or an isopropyl group.

**30.** The use according to claim 27, wherein R in the formula (1) is an ethyl group.

[Fig. 1]

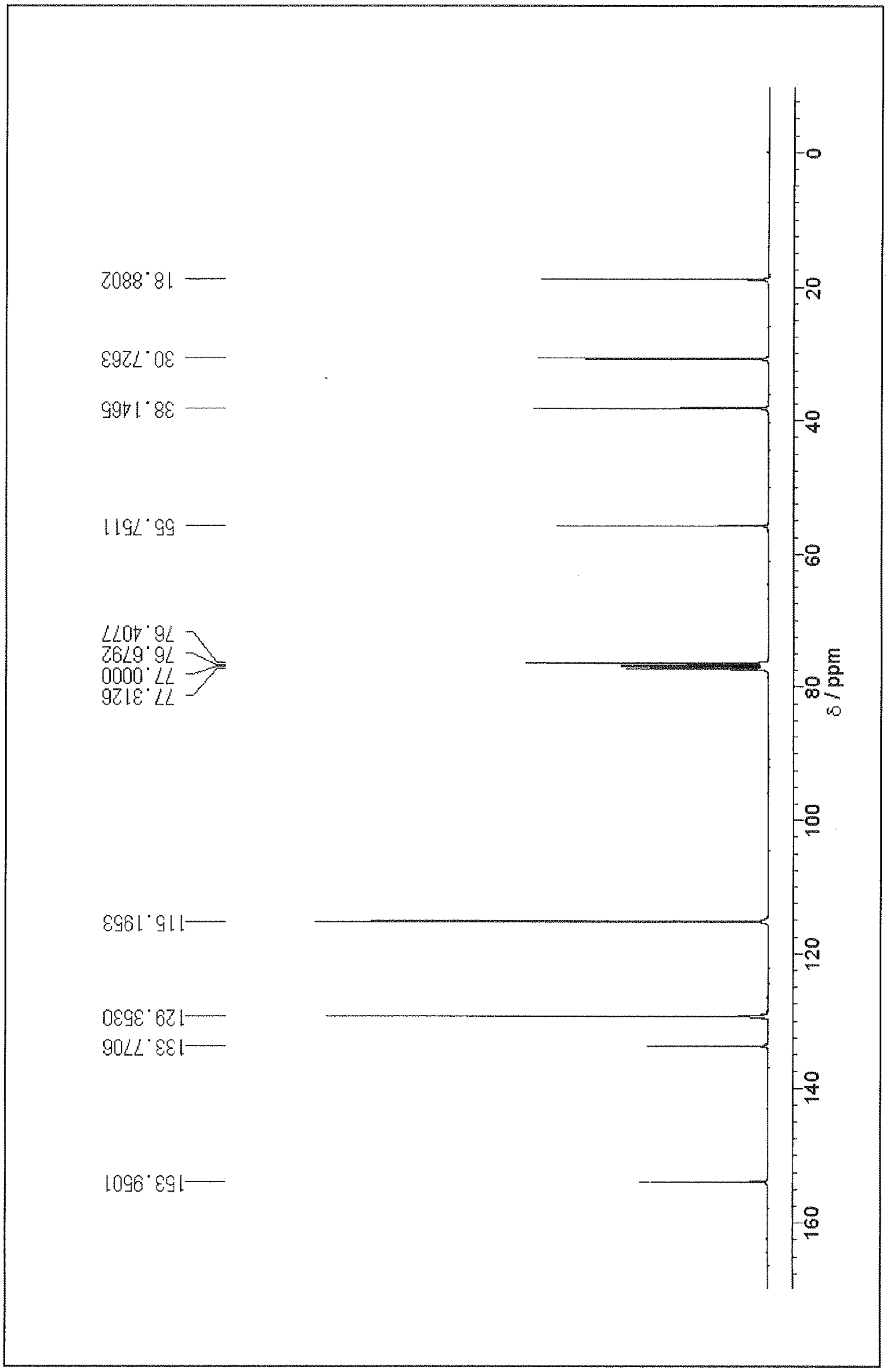

153.9601
133.7706
129.3630
115.1953
77.3126
77.0000
76.6792
76.4077
55.7511
38.1465
30.7263
18.8802
160
140
120
100
80
δ / ppm
60
40
20
0

[Fig. 2]

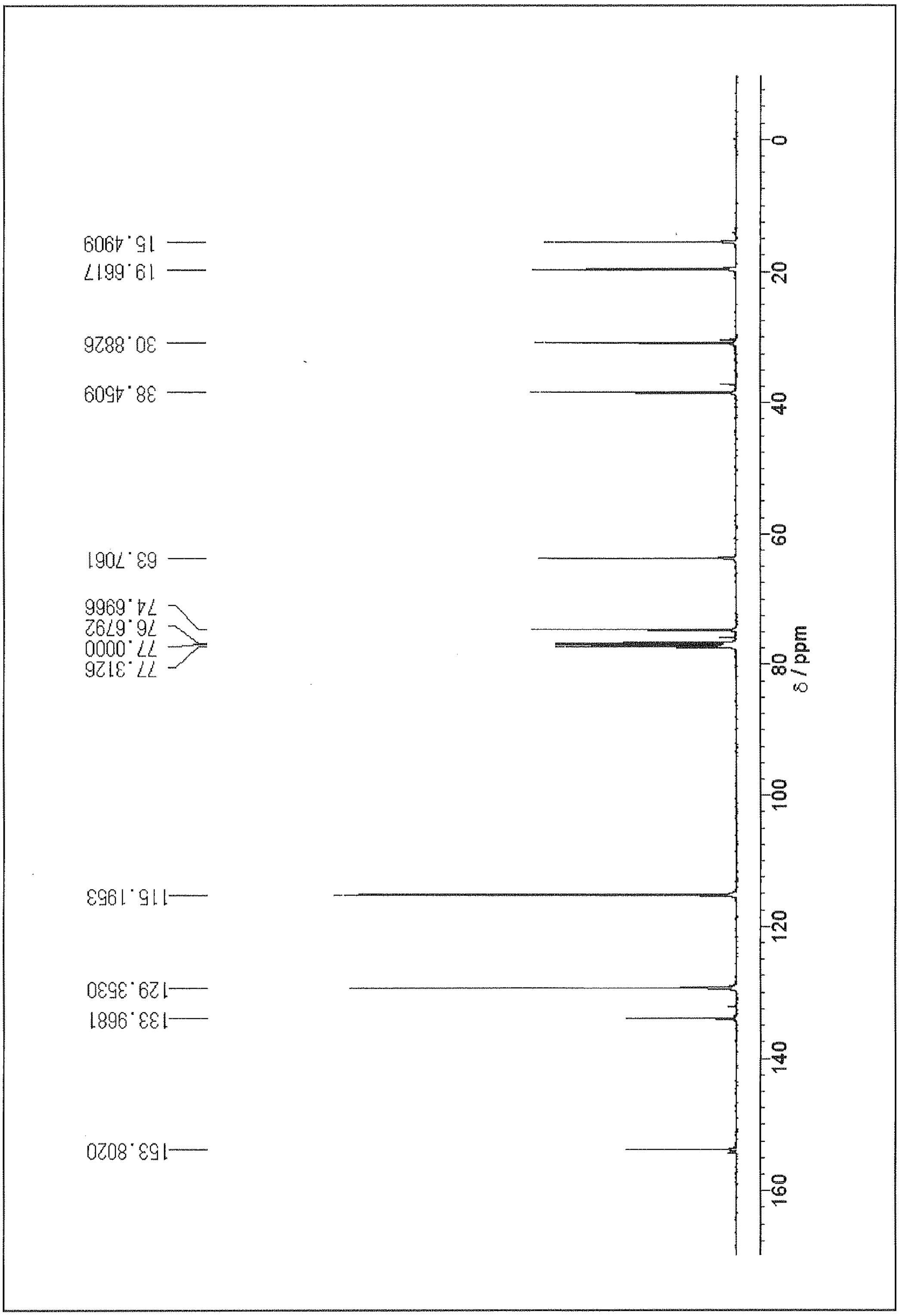
15.4909
19.6617
30.8826
38.4609
63.7061
74.6966
76.6792
77.0000
77.3126
115.1953
129.3530
133.9681
153.8020
0
20
40
60
80
100
120
140
160
δ / ppm

[Fig. 3]

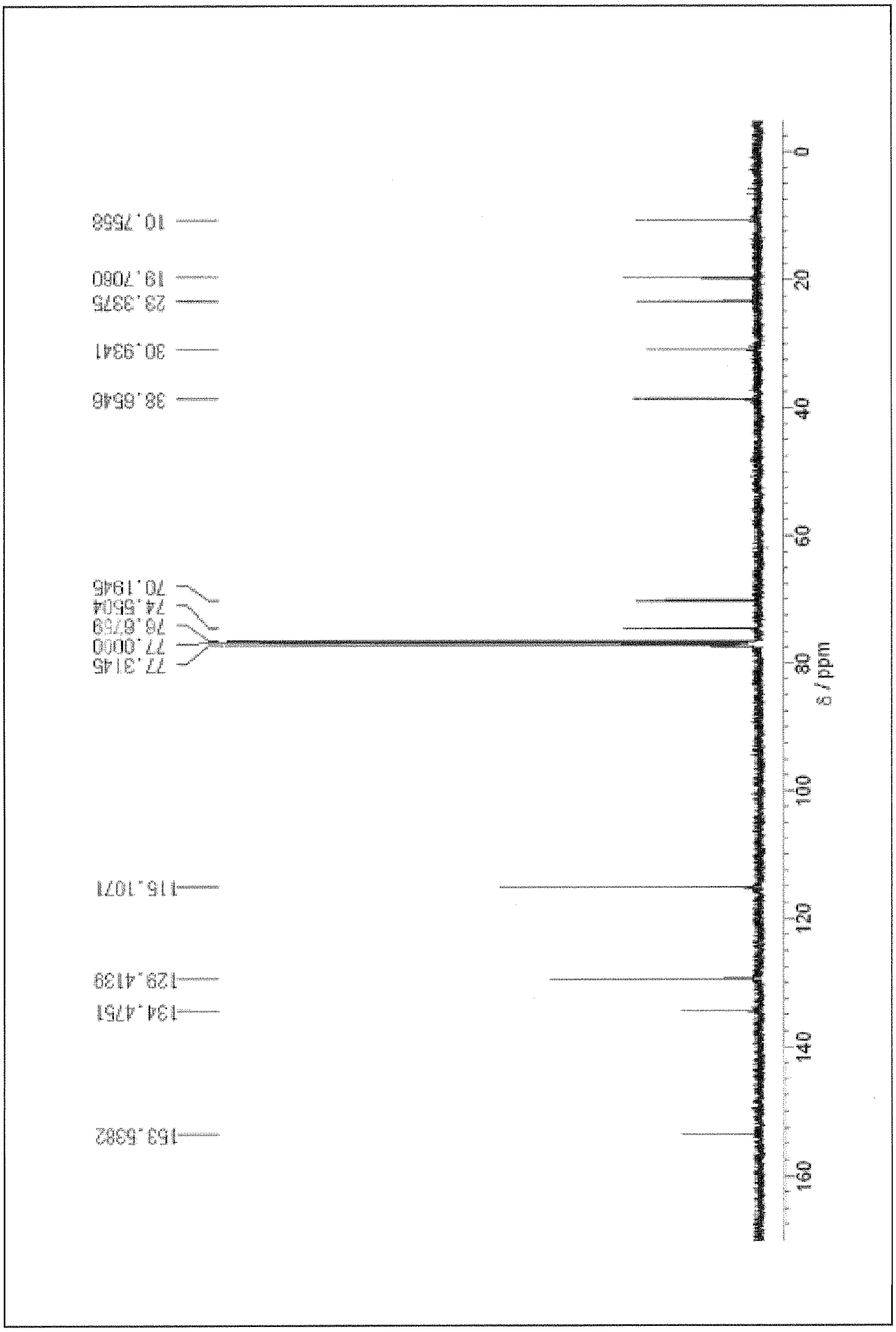
10.7558
19.7080
23.3375
30.9341
38.6546
70.1946
74.5504
76.6759
77.0000
77.3145
115.1071
129.4139
134.4751
153.5382
0
20
40
60
80
100
120
140
160
δ / ppm

[Fig. 4]

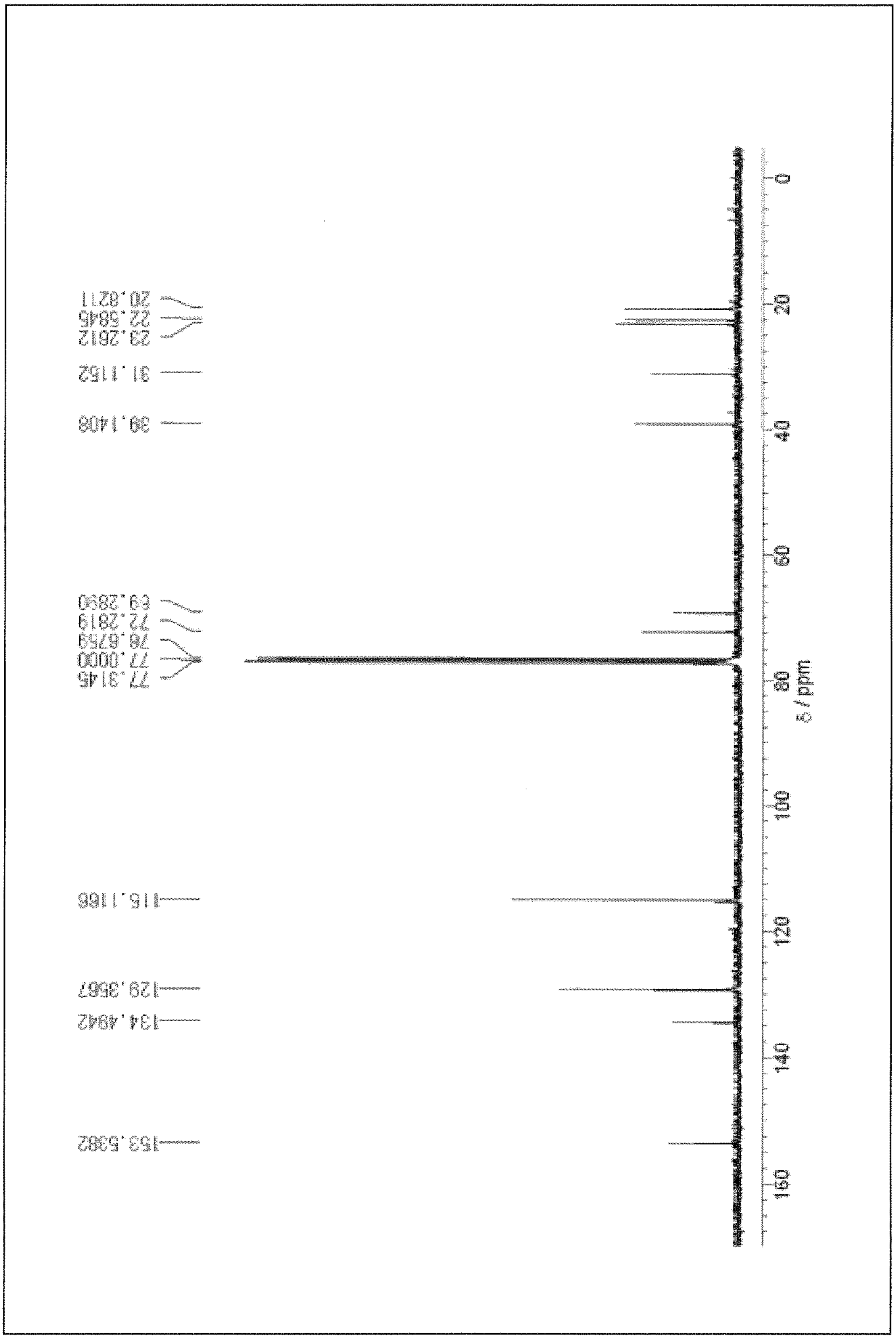
20.8211
22.5845
23.2612
31.1152
39.1408
69.2890
72.2819
76.6759
77.0000
77.3145
115.1166
129.3567
134.4942
153.5382
0
20
40
60
80
100
120
140
160
δ / ppm

[Fig. 5]

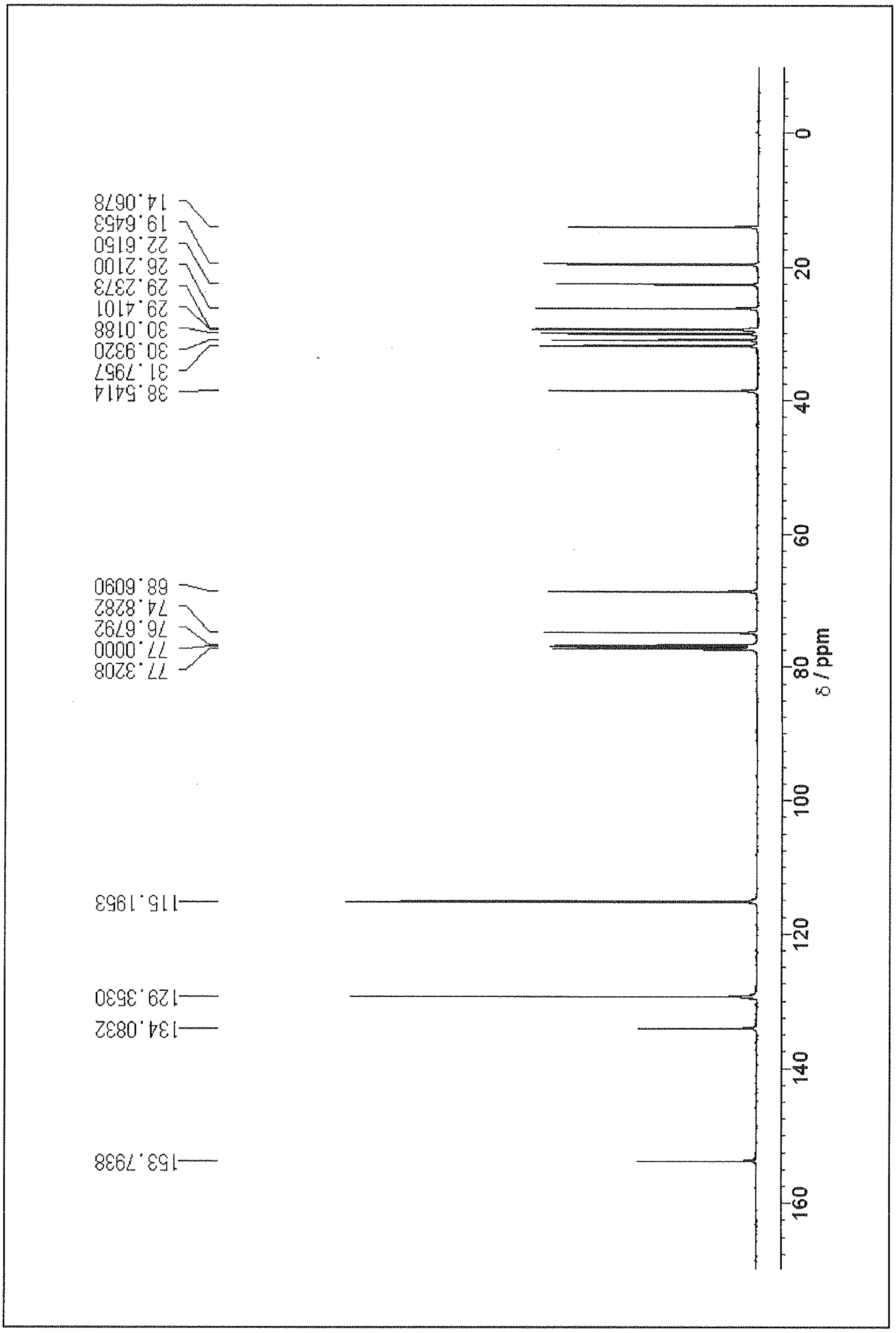
14.0678
19.6453
22.6150
26.2100
29.2373
29.4101
30.0188
30.9920
31.7957
38.5414
68.6090
74.8282
76.6792
77.0000
77.3208
115.1953
129.3630
134.0832
153.7938
0
20
40
60
80
100
120
140
160
δ / ppm

**INTERNATIONAL SEARCH REPORT**

International application No.
PCT/JP2011/058854

A. CLASSIFICATION OF SUBJECT MATTER
*A61K8/34*(2006.01)i, *A61Q19/02*(2006.01)i

According to International Patent Classification (IPC) or to both national classification and IPC

B. FIELDS SEARCHED

Minimum documentation searched (classification system followed by classification symbols)
A61K8/34, A61Q19/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 02-188547 A (Sumitomo Chemical Co., Ltd.), 24 July 1990 (24.07.1990), claims; particularly, claim 3; page 10, upper right column, line 8 to page 11, upper right column, line 7; tables 1, 2<br>& US 5389293 A & EP 0806417 A1 | 13-18<br>1-12,19-30 |
| X<br>A | JP 03-047143 A (Sumitomo Chemical Co., Ltd.), 28 February 1991 (28.02.1991), claims; examples 13 to 16, 21 to 24; tables 13, 15<br>(Family: none) | 13-18<br>1-12,19-30 |
| A | JP 10-265325 A (Kanebo, Ltd.), 06 October 1998 (06.10.1998), claims; examples<br>& TW 464501 B | 1-30 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
05 July, 2011 (05.07.11)

Date of mailing of the international search report
12 July, 2011 (12.07.11)

Name and mailing address of the ISA/
Japanese Patent Office

Facsimile No.

Authorized officer

Telephone No.

Form PCT/ISA/210 (second sheet) (July 2009)

**INTERNATIONAL SEARCH REPORT**

International application No.
PCT/JP2011/058854

C (Continuation). DOCUMENTS CONSIDERED TO BE RELEVANT

| Category* | Citation of document, with indication, where appropriate, of the relevant passages | Relevant to claim No. |
|---|---|---|
| A | JP 06-107539 A (Marine Biotechnology Institute Co., Ltd.), 19 April 1994 (19.04.1994), claims; paragraphs [0006], [0014] (Family: none) | 1-30 |
| A | JP 2008-100953 A (Kao Corp.), 01 May 2008 (01.05.2008), claims; paragraphs [0001], [0022]; preparation example 1; examples 1 to 3 (Family: none) | 1-30 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10265325 A
- JP 2188547 A


### Non-patent literature cited in the description


- **MICHAEL et al.** *Journal of the American Chemical Society,* 1972, 3659-3661